# EUROPEAN PATENT APPLICATION

(11) **EP 2 103 612 A1**
(43) Date of publication of application: **23.09.2009**
(21) Application number: 09007479.0
(22) Date of filing: 23.10.2007
(51) Int. Cl.: C07D 453/02, A61K 31/473, A61P 1/08

(54) **Crystalline forms of palonosetron hydrochloride**

(30) Priority: 23.10.2006 US 853840 P; 28.11.2006 US 861488 P; 29.11.2006 US 861847 P; 01.02.2007 US 899102 P; 01.02.2007 US 899109 P; 20.03.2007 US 919165 P; 14.08.2007 US 955679 P
(62) Divisional of application: 07839759.3
(71) Applicant: Sicor, Inc., Irvine, CA 92618 (US)
(72) Inventor: Rossetto, Pierluigi, Balerna (CH); Macdonald, Peter, Lindsay, Gentilino (CH); Banfi, Gaia, Lentate Sul Seveso (MI) (IT); Nemethne, Racz Csilla, 4440 Tiszavasvari (HU)
(74) Representative: King, Lawrence

(57) **Abstract**

The present invention provides crystalline forms of Palonosetron hydrochloride, processes for their preparation and pharmaceutical compositions containing such crystalline forms of Palonosetron HCl.

## Description

### CROSS REFERENCE TO RELATED APPLICATIONS

The present application claims the benefit of the following United States Provisional Patent Application Nos.: 60/853,840, filed October 23, 2006; 60/861,488, filed November 28, 2006; 60/861,847, filed November 29, 2006; 60/899,102, filed February 1,2007; 60/899,109, filed February 1, 2007; 60/919,615, filed March 20, 2007; and 60/955,679, filed August 14,2007. The contents of these applications are incorporated herein by reference.

### FILED OF INVENTION

The present invention is directed to new crystalline forms of Palonosetron hydrochloride, and processes for preparation thereof and pharmaceutical compositions thereof.

### BACKGROUND OF INVENTION

Palonosetron hydrochloride, 1H-Benz[de]isoquinolin-1-one, 2-(3S)-1-azabicyclo[2.2.2]oct-3-yl-2,3,3aS,4,5,6-hexahydro- monohydrochloride, of the following formula: 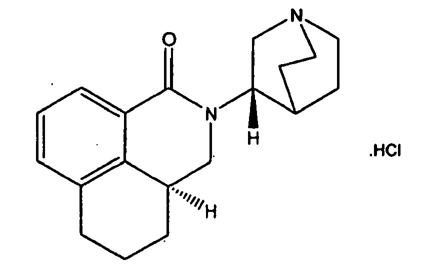 is a selective 5HT3-antagonist (prevention of chemotherapy-induced nausea and vomiting). It is marketed as a solution, under the name Aloxi^{®} by Hellsinn.

Palonosetron hydrochloride and its preparation were first reported in US patent No. 5,202,333. Also, reported is the isolation of Palonosetron hydrochloride by crystallization from ethanol, providing Palonosetron hydrochloride with a melting point of 296-297°C.

Crystallization of Palonosetron hydrochloride is also reported in US patent No. 5,567,818. The process involves subjecting 1078.8 g of solid comprising a diastereomeric mixture of 97% 3aS and 3% 3aR 2-(1-azabicyclo[2.2.2]ocy-3S-yl) 2,3,3a,4,5,6-hexahydro-1-H-benz[de]isoquinolin-1-one hydrochloride to a processas follows: "The diastereomeric mixture ... was dissolved in 29.3 L of isopropanol. The solution was heated to reflux and 1 L of water and 2.5 L of additional isopropanol was added. The mixture was distilled to a volume of approximately 16 L, cooled over 2 hours to 20°C. and then cooled to 5°C. and stirred for approximately 18 hours giving a crystalline precipitate. The precipitate was isolated by filtration and dried in a nitrogen/vacuum oven at 68°C."

The occurrence of different solid state forms (polymorphism) is a property of some molecules and molecular complexes. A single molecule, like the Palonosetron hydrochloride in the above formula may give rise to a variety of solids having distinct physical properties like melting point, X-ray diffraction pattern, infrared absorption fingerprint and NMR spectrum. The differences in the physical properties of polymorphs result from the orientation and intermolecular interactions of adjacent molecules (complexes) in the bulk solid. Accordingly, polymorphs are distinct solids sharing the same molecular formula yet having distinct advantageous and/or disadvantageous physical properties compared to other forms in the polymorph family. One of the most important physical properties of pharmaceutical polymorphs is their solubility in aqueous solution.

The discovery of new polymorphic forms of a drug enlarges the repertoire of materials that a formulation scientist has available with which to design a pharmaceutical dosage form of a drug with a targeted release profile or other desired characteristics. Therefore, there is a need to find more crystalline forms of Palonosetron hydrochloride.

Thus, there is a need in the art for new crystalline forms of Palonosetron hydrochloride and processes for the preparation thereof.

### SUMMARY OF INVENTION

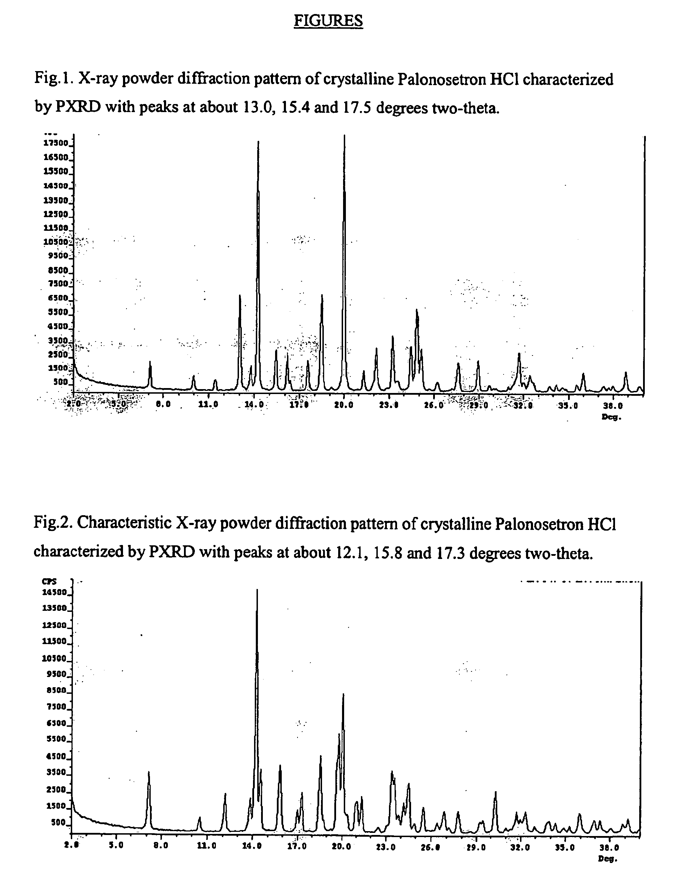

In one aspect, the present invention provides crystalline form of Palonosetron hydrochloride characterized by data selected from the group consisting of a PXRD having peak reflections at about 13.0,15.4 and 17.5 ±0.2 degrees two-theta, a PXRD substantially as depicted in figure 1, and a combination thereof.

In another aspect, the present invention provides a process for preparing crystalline Palonosetron hydrochloride characterized by data selected from the group consisting of a PXRD having peak reflections at about 13.0, 15.4 and 17.5 ±0.2 degrees two-theta, a PXRD substantially as depicted in figure 1, and a combination thereof, comprising crystallizing Palonosetron hydrochloride having less than 65% of Palonosteron 3aS Palonostreon HCl of the following formula, 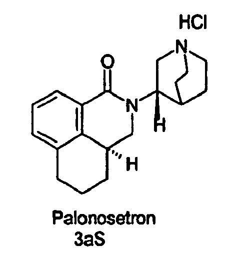 from a solvent selected from the group consisting of methanol, a mixture of isoproanol and water, and a mixture of methanol, isoproanol and water at a temperature below 55°C.

In another aspect, the present invention provides crystalline form of Palonosetron hydrochloride characterized by data selected form the group consisting of a PXRD having peak reflections at about 12.1, 15.8 and 17.3 ±0.2 degrees two-theta, a PXRD substantially as depicted in figure 2, and a combination thereof.

In another aspect, the present invention provides a process for preparing crystalline Palonosetron hydrochloride characterized by data selected form the group consisting of a PXRD having peak reflections at about 12.1, 15.8 and 17.3 ±0.2 degrees two-theta, a PXRD substantially as depicted in figure 2, and a combination thereof, comprising crystallizing Palonosetron hydrochloride having at least 96% of Palonosteron 3aS Palonostreon HCl of the following formula, 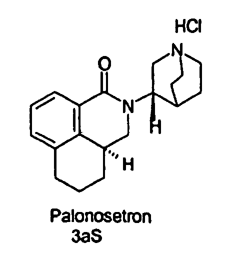 from a mixture of isopropanol and water at a temperature above 55°C.

In yet another aspect, the present invention provides another process for preparing crystalline Palonosetron hydrochloride characterized data selected form the group consisting of a PXRD having peak reflections at about 12.1, 15.8 and 17.3 ±0.2 degrees two-theta, a PXRD substantially as depicted in figure 2, and a combination thereof, comprising exposing crystalline Palonosetron hydrochloride characterized by data selected from the group consisting of: a PXRD having peak reflections at about 13.0, 15.4 and 17.5 ±0.2 degrees two-theta, a PXRD substantially as depicted in figure 1, and a combination thereof to relative humidity of 100%.

In yet another aspect, the present invention provides a pharmaceutical composition comprising at least one of the crystalline forms of Palonosetron hydrochloride of the present invention and a pharmaceutically acceptable excipient.

In one aspect, the present invention provides a process for preparing a pharmaceutical composition comprising at least one of the crystalline forms of Palonosetron hydrochloride of the present invention and a pharmaceutically acceptable excipient.

In one embodiment, the present invention provides pharmaceutical formulations comprising at least one of the crystalline forms of Palonosetron hydrochloride prepared according to the processes of the present invention, and a pharmaceutically acceptable excipient.

In another embodiment, the present invention provides a process for preparing pharmaceutical formulations comprising at least one of the crystalline forms of Palonosetron hydrochloride prepared according to the processes of the present invention, and a pharmaceutically acceptable excipient.

### BRIEF DESCRIPTION OF THE FIGURES

Fig.1 illustrates X-ray powder diffraction pattern of crystalline Palonosetron HCl characterized by data selected from the group consisting of PXRD with peaks at about 13.0, 15.4 and 17.5 degrees two-theta, a PXRD substantially as depicted in figure 1, and a combination thereof.
Fig.2. illustrates X-ray powder diffraction pattern of crystalline Palonosetron HCl characterized by data selected from the group consisting of: a PXRD with peaks at about 12.1, 15.8 and 17.3 degrees two-theta, a PXRD figure as depicted in figure 2, and a combination thereof.

### DETAILED DESCRIPTION OF INVENTION

The present invention provides novel crystalline forms of Palonosetron hydrochloride (referred to as PAL-HCl), processes for making them, and pharmaceutical compositions thereof.

The present invention provides crystalline form of PAL-HCl characterized by data selected from the group consisting of a PXRD having peak reflections at about 13.0, 15.4 and 17.5 ±0.2 degrees two-theta, a PXRD pattern substantially as depicted in figure 1, and a combination thereof.

The said crystalline form can be further characterized by a PXRD with peaks at about 7.1, 13.7, 14.2, 16.2, 18.5, 20.0 and 22.1 ±0.2 degrees two-theta. The said crystalline form of PAL-HCl can also be characterized by a DSC thermogram having an endothermic peak at about 311°C due to melting.

In addition, the above crystalline form can be characterized by a weight loss of less than about 0.1 % measured at temperatures less or equal to 153°C by TGA. In addition, the above crystalline form is an anhydrous form of PAL-HCl.

The said crystalline form of PAL-HCl has less than 20%, preferably less than 10%, more preferably less than 5% of the crystalline Palonosetron hydrochlroide characterized by data selected from the group consisting of: a PXRD having peak reflections at about 12.1, 15.8 and 17.3 ±0.2 degrees two-theta, a PXRD pattern substantially as depicted in figure 2, and a combination thereof. Typically, the content of crystalline Palonosetron hydrochlroide characterized by data selected from the group consisting of: a PXRD having peak reflections at about 12.1, 15.8 and 17.3 ±0.2 degrees two-theta, a PXRD pattern substantially as depicted in figure 2, and a combination thereof is measured by % by weight, preferably as determined by PXRD.

The above crystalline PAL-HCl is prepared by a process comprising crystallizing Palonosetron hydrochloride having less than 65% of 3aS Palonosetron HCl of the following formula, 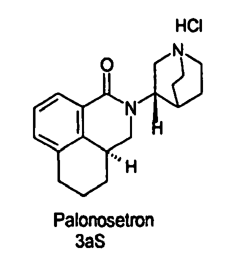 from a solvent selected from the group consisting of: methanol, a mixture of isopropanol and water, and a mixture of methanol, isopropanol and water, at a temperature below 55°C.

The crystallization process comprises providing a solution of PAL-HCl having less than 65% of 3aS Palonosetron HCl in a solvent selected from the group consisting: methanol, a mixture of isopropanol and water, and a mixture of methanol, isopropanol and water, and precipitating the said crystalline PAL-HCl at a temperature below 55°C.

Preferably, the content of the 3aS Palonostreon HCl isomer in Palonostreon HCl is of about 65% to about 50%. The measurement of the content of the 3aS Palonostreon HCl isomer in Palonostreon HCl is by % area, preferably, by HPLC.

The above solution is preferably, prepared by a process comprising combining PAL-HCl having less than 65% of 3aS Palonostreon HCl and a solvent at a temperature of about 25°C to about reflux to obtain said solution.

In a preferred embodiment the solution of PAL-HCl in methanol is provided by combining PAL-HCl and methanol at a temperature of about 10°C to about 30°C, preferably of about 20°C to about 25°C, more preferably of about 25°C.

In another preferred embodiment, the solution of PAL-HCl in a mixture of IPA and water is provided by combining PAL-HCl having less than 65% of 3aS Palonosetron HCl and a mixture of IPA and water at a temperature of at least about 75°C.

In yet another preferred embodiment, the solution of PAL-HCl in a mixture of methanol, IPA and water is provided by combining a methanolic residue of PAL-HCl having less than 65% of 3aS Palonosetron HCl with a mixture of IPA and water at a temperature of at least 75°C.

Preferably, the methanolic residue of PAL-HCl having less than 65% of 3aS Palonosetron HCl can be prepared by a process comprising concentrating a solution of PAL-HCl having less than 65% of 3aS Palonosetron HCl in methanol to obtain a concentrated solution, adding IPA or a mixture of IPA and water to obtain a mixture, concentrating the mixture to obtain said methanolic residue. This process may be repeated.

Preferably, the ratio of the solvents in the mixture of IPA-water is of about 94:6 to about 97:3, more preferably, of about 95:5 to about 97:3, respectively.

Preferably, combining PAL-HCl having less than 65% of 3aS Palonosetron HCl or a methanolic residue thereof with a mixture of IPA and water is done at about 75°C to about 90°C, more preferably at about 77°C to about 85°C, even more preferably, at about 77°C to about 80°C, most preferably, at reflux temperature. The reflux temperature may vary according to the total amount of water. In this process of the present invention, water can be present in the solution in a total amount of about 2 to about 6% V/V. The solution obtained in this process of the present invention may comprise methanol, IPA and water in a ratio of about 0.5:94.5:5 to about 0.1:94.9:5 V/V, respectively.

Precipitation can be achieved by cooling the solution, by evaporating the solvent or by combination of both, wherein both are done at a temperature below 55°C. When methanol is the solvent, the precipitation is achieved, preferably, by evaporating the solution at a temperature of about 10°C to about 30°C, more preferably at about 20°C to about 25°C, even more preferably of about 25°C.

When a mixture of IPA and water is the solvent, the precipitation is achieved, preferably, by cooling the solution at a temperature below about 55°C in order to obtain a suspension, preferably, to a temperature of about 48-52°C, then the temperature is further decreased to about 0°C, more preferably, to a temperature 25°C to about 0°C, to obtain a suspension, increasing the recovery yield.

The amount of precipitated crystalline product may be increased by a process comprising at least one of the following steps: concentrating the second suspension, and further maintaining the second suspension at such cooled temperature for a sufficient period of time. Preferably, concentration is carried out at a temperature of about 45°C to about 55°C. Preferably, the suspension is further maintained at a temperature of about 25°C to about 0°C, more preferably, of about 10°C to about 0°C. Preferably, the second suspension is maintained for about 10 to about 24 hours, more preferably, for about 15 to about 20 hours.

The process for preparing the above characterized crystalline PAL-HCl may further comprise a recovery process. This crystalline PAL-HCl may be recovered by any process known to a skilled artisan, such as filtering and/or drying.

The present invention also provides crystalline PAL-HCl characterized by data selected from the group consisting of a PXRD having peak reflections at about 12.1, 15.8 and 17.3 ±0.2 degrees two-theta, a PXRD pattern substantially as depicted in figure 2, and a combination thereof.

This crystalline can be further characterized by said PXRD pattern with peaks at about 7.1, 13.8, 14.2, 14.5, 18.5, 20.0 and 30.3 ±0.2 degrees two-theta.

Said crystalline PAL-HCl can be further characterized by a DSC thermogram having an endothermic peak at about 313°C due to melting. In addition, the above crystalline PAL-HCl can be characterized by a weight loss of less than about 0.1% measured at temperatures less or equal to 152°C by TGA. In addition, the above crystalline PAL-HCl is an anhydrous form of PAL-HCl.

This crystalline PAL-HCl has less than 20%, preferably, less than 10%, more preferably, less than 5% of crystalline Palonosetron hydrochlroide characterized by data selected from the group consisting of a PXRD having peak reflections at about 13.0, 15.4 and 17.5 ±0.2 degrees two-theta, a PXRD pattern substantially as depicted in figure 1, and a combination thereof. The content of crystalline Palonosetron hydrochlroide characterized by data selected from the group consisting of a PXRD having peak reflections at about 13.0, 15.4 and 17.5 ±0.2 degrees two-theta, a PXRD pattern substantially as depicted in figure 1, and a combination thereof may be measured by % by weight, preferably, by PXRD.

The crystalline PAL-HCl data selected from the group consisting of: a PXRD having peak reflections at about 12.1, 15.8 and 17.3 ±0.2 degrees two-theta, a PXRD pattern substantially as depicted in figure 2, and a combination thereof is prepared by a process comprising crystallizing Palonosetron hydrochloride having at least 96% of 3aS Palonosetron HCl of the following formula, 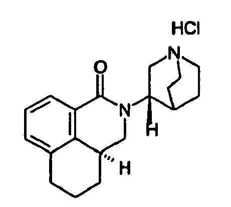 from a mixture of isopropanol and water at a temperature above 55°C.

In one embodiment, crystallization is carried out by providing a solution of PAL-HCl having at least 96% of 3aS Palonostreon HCl, in a mixture comprising IPA and water at a temperature above 55°C, and precipitating the said crystalline PAL-HCl at a temperature of at least about 55°C.

The measurement of the content of the 3aS Palonostreon HCl isomer in Palonostreon HCl is by % area, preferably, by HPLC.

The solution is provided by a process comprising admixing PAL-HCl having at least 96% of 3aS Palonostreon HCl with a mixture comprising IPA and water to obtain a suspension, and heating the suspension to a temperature above 55°C.

The starting PAL-HCl may be a dry solid. The term "dry" in reference to PAL-HCl, as used herein, refers to a solid that doesn't lose any weight when being dried.

Preferably, the ratio of the solvents in the mixture of IPA-water is of about 94:6 to about 98:2, more preferably, of about 95:5 to about 98:2, respectively.

Preferably, the suspension is heated to a temperature of at least 75°C, preferably, at about 75°C to about 90°C, more preferably about 77°C to about 85°C, even more preferably, at about 77°C to about 80°C, most preferably, at reflux temperature. The reflux temperature may vary according to the total amount of water. In this process of the present invention, water can be present in the solution in a total amount of about 2 to about 6% V/V.

In the above process precipitation is achieved by cooling the solution to a temperature of at least about 55°C, preferably about 58°C to about 60°C, to obtain a suspension. The amount of precipitated crystalline product may be increased by a process comprising at least one of the following steps: cooling the obtained suspension, concentrating the obtained suspension, and further maintaining the second suspension at such temperature for a sufficient period of time, or any combination thereof.

Preferably, concentration is carried out at a temperature of about 50°C to about 40°C. Preferably, the suspension is further maintained at a temperature of about 20°C to about 0°C. Preferably, the second suspension is maintained for about 10 to about 18 hours, more preferably, for about 30 min to about 18 hours.

The process for preparing the above crystalline PLA-HCl may further comprise a recovery process. The said crystalline form may be recovered by any process known to a skilled artisan, such as filtering and drying.

Crystalline Palonosetron hydrochloride characterized by data selected form the group consisting of a PXRD having peak reflections at about 12.1, 15.8 and 17.3 ±0.2 degrees two-theta, a PXRD pattern substantially as depicted in figure 2, and a combination thereof can be prepared by another process comprising exposing crystalline Palonosetron hydrochloride characterized by data selected from the group consisting of: a PXRD having peak reflections at about 13.0, 15.4 and 17.5 ±0.2 degrees two-theta, a PXRD pattern substantially as depicted in figure 1, and a combination thereof to relative humidity of 100%.

Preferably, the starting crystalline PAL-HCl is exposed to relative humidity of 100% at a temperature of about 20°C to about 30°C (room temperature), for a period of about 2 days to about 10 days, preferably about 4 to about 8 days, and more preferably about 7 days.

The starting PAL-HCl for preparing any of the crystalline forms of PAL-HCL of the present invention can be obtained, for example, according to the processes described in co-pending application No. 11/...,... (Attorney Docket 13150/48504), filed October 23, 2007.

The process comprises reacting Cp 9588 of the following formula, 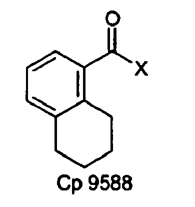 with a salt of Cp 9771 of the following formula 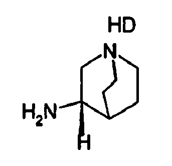 and a base in a solvent mixture comprising water and a water immiscible organic solvent to obtain the free base of Cp 9563 of the following formula; 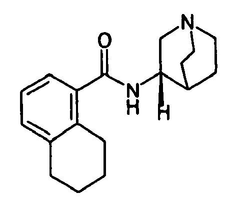 recovering the free base of Cp 9563; reacting the free base of Cp 9563 with a lithium base, with DMF and with an acid, preferably HCl, to obtain Cp 9558 of the following formula; 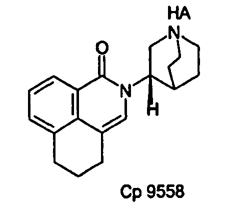 recovering Cp 9558; and reacting Cp 9558 in an alcohol with not more than 20% of hydrogenation catalyst per gram of Cp 9558, to obtain Palonosetron salt; wherein HA and HD each are an acid, preferably, HCl.

In another process described therein Cp 9588 is reacted with Cp 9771 to obtain the salt Cp 9563 of the following formula 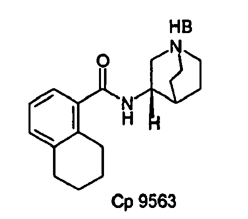 which is reacted with a lithium base, with DMF and with an acid, preferably HCl, to obtain Cp 9558; and reacting Cp 9558 in an alcohol with not more than 20% of hydrogenation catalyst per gram of Cp 9558, to obtain Palonosetron salt, wherein HB is an acid, preferably HCl.

The crystalline forms of the present invention can be used in a pharmaceutical composition for treatment for prevention of chemotherapy-induced nausea and vomiting.

The present invention provides a pharmaceutical composition comprising at least one of the crystalline forms of Palonosetron hydrochloride of the present invention and a pharmaceutically acceptable excipient.

The present invention also provides a process for preparing a pharmaceutical composition comprising at least one of the crystalline forms of Palonosetron hydrochloride of the present invention and a pharmaceutically acceptable excipient.

Methods of administration of a pharmaceutical composition of the present invention can be administered in various preparations depending on the age, sex, and symptoms of the patient. The pharmaceutical compositions can be administered, for example, as tablets, pills, powders, liquids, suspensions, emulsions, granules, capsules, suppositories, injection preparations (solutions and suspensions), and the like.

Pharmaceutical compositions of the present invention can optionally be mixed with other forms ofPalonosetron hydrochloride and/or other active ingredients. In addition, pharmaceutical compositions of the present invention can contain inactive ingredients such as diluents, carriers, fillers, bulking agents, binders, disintegrants, disintegration inhibitors, absorption accelerators, wetting agents, lubricants, glidants, surface active agents, flavoring agents, and the like.

Diluents increase the bulk of a solid pharmaceutical composition and can make a pharmaceutical dosage form containing the composition easier for the patient and care giver to handle. Diluents for solid compositions include, for example, microcrystalline cellulose (e.g., Avicel^{®}), microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., Eudragit^{®}), potassium chloride, powdered cellulose, sodium chloride, sorbitol, or talc.

Carriers for use in the pharmaceutical compositions may include, but are not limited to, lactose, white sugar, sodium chloride, glucose, urea, starch, calcium carbonate, kaolin, crystalline cellulose, or silicic acid.

Bindery help bind the active ingredient and other excipients together after compression. Binders for solid pharmaceutical compositions include for example acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucelfi, hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate, or starch.

Disintegrants can increase dissolution. Disintegrants include, for example, alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

Disintegration inhibitors may include, but are not limited to, white sugar, stearin, coconut butter, hydrogenated oils, and the like.

Absorption accelerators may include, but are not limited to, quaternary ammonium base, sodium laurylsulfate, and the like.

Wetting agents may include, but are not limited to, glycerin, starch, and the like. Adsorbing agents used include, but are not limited to, starch, lactose, kaolin, bentonite, colloidal silicic acid, and the like.

A lubricant can be added to the composition to reduce adhesion and ease release of the product from a punch or dye during tableting. Lubricants include for example magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Glidants can be added to improve the flowability of non-compacted solid composition and improve the accuracy of dosing. Excipients that can function as glidants include for example colloidal silicon dioxide, magnesium trisilicate, powdered cellulose, starch, talc and tribasic calcium phosphate.

Flavoring agents and flavor enhancers make the dosage form more palatable to the patient. Common flavoring agents and flavor enhancers for pharmaceutical products that can be included in the composition of the present invention include for example maltol, vanillin, ethyl vanillin, menthol, citric acid, fumaric acid, ethyl maltol, and tartaric acid.

Tablets can be further coated with commonly known coating materials such as sugar coated tablets, gelatin film coated tablets, tablets coated with enteric coatings, tablets coated with films, double layered tablets, and multi-layered tablets. Capsules can be coated with shell made, for example, from gelatin and optionally contain a plasticizer such as glycerin and sorbitol, and an opacifying agent or colorant.

Solid and liquid compositions can also be dyed using any pharmaceutically acceptable colorant to improve their appearance and/or facilitate patient identification of the product and unit dosage level.

In liquid pharmaceutical compositions of the present invention, the Palonosetron hydrochloride of the present invention is suspended, while maintaining its crystalline characteristics, and any other solid ingredients are dissolved or suspended in a liquid carrier, such as water, vegetable oil, alcohol, polyethylene glycol, propylene glycol or glycerin.

Liquid pharmaceutical compositions can contain emulsifying agents to disperse uniformly throughout the composition an active ingredient or other excipient that is not soluble in the liquid carrier. Emulsifying agents that can be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention can also contain viscosity enhancing agents to improve the mouth-feel of the product and/or coat the lining of the gastrointestinal tract. Such agents include for example acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar can be added to improve the taste. Preservatives and chelating agents such as alcohol, sodium benzoate, butylated hydroxy toluene, butylated hydroxyanisole and ethylenediamine tetraacetic acid can be added at safe levels to improve storage stability.

A liquid composition according to the present invention can also contain a buffer such as guconic acid, lactic acid, citric acid or acetic acid, sodium guconate, sodium lactate, sodium citrate or sodium acetate.

Selection of excipients and the amounts to use can be readily determined by an experienced formulation scientist in view of standard procedures and reference works known in the art.

A composition for tableting or capsule filing can be prepared by wet granulation. In wet granulation some or all of the active ingredients and excipients in powder form are blended and then further mixed in the presence of a liquid, typically water, which causes the powders to clump up into granules. The granulate is screened and/or milled, dried and then screened and/or milled to the desired particle size. The granulate can then be tableted or other excipients can be added prior to tableting, such as a glidant and/or a lubricant.

A tableting composition can be prepared conventionally by dry blending. For instance, the blended composition of the actives and excipients can be compacted into a slug or a sheet and then comminuted into compacted granules. The compacted granules can be compressed subsequently into a tablet.

As an alternative to dry granulation, a blended composition can be compressed directly into a compacted dosage form using direct compression techniques. Direct compression produces a more uniform tablet without granules. Excipients that are particularly well-suited to direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention can comprise any of the aforementioned blends and granulates that were described with reference to tableting, only they are not subjected to a final tableting step.

When shaping the pharmaceutical composition into pill form, any commonly known excipient used in the art can be used. For example, carriers include, but are not limited to, lactose, starch, coconut butter, hardened vegetable oils, kaolin, talc, and the like. Binders used include, but are not limited to, gum arabic powder, tragacanth gum powder, gelatin, ethanol, and the like. Disintegrating agents used include, but are not limited to, agar, laminalia, and the like.

For the purpose of shaping the pharmaceutical composition in the form of suppositories, any commonly known excipient used in the art can be used. For example, excipients include, but are not limited to, polyethylene glycols, coconut butter, higher alcohols, esters of higher alcohols, gelatin, semisynthesized glycerides, and the like.

When preparing injectable pharmaceutical compositions, solutions and suspensions are sterilized and are preferably made isotonic to blood. Injection preparations may use carriers commonly known in the art. For example, carriers for injectable preparations include, but are not limited to, water, ethyl alcohol, propylene glycol, ethoxylated isostearyl alcohol, polyoxylated isostearyl alcohol, and fatty acid esters of polyoxyethylene sorbitan. One of ordinary skill in the art can easily determine with little or no experimentation the amount of sodium chloride, glucose, or glycerin necessary to make the injectable preparation isotonic. Additional ingredients, such as dissolving agents, buffer agents, and analgesic agents may be added. If necessary, coloring agents, preservatives, perfumes, seasoning agents, sweetening agents, and other medicines may also be added to the desired preparations during the treatment of schizophrenia.

The amount of Palonosetron hydrochloride of the present invention contained in a pharmaceutical composition according to the present invention is not specifically restricted; however, the dose should be sufficient to treat, ameliorate, or reduce the condition.

Having described the invention with reference to certain preferred embodiments, other embodiments will become apparent to one skilled in the art from consideration of the specification. The invention is further defined by reference to the following examples describing in detail the process and compositions of the invention. It will be apparent to those skilled in the art that many modifications, both to materials and methods, may be practiced without departing from the scope of the invention.

### EXAMPLES

### PXRD method

ARL X-ray powder diffractometer model X'TRA-030, Peltier detector, round standard aluminium sample holder with round zero background quartz plate was used. The cathode is CuK_{α} radiation, λ = 1.5418 A. Scanning parameters: Range: 2-40 deg. 2θ, continuous Scan, Rate: 3 deg./min. The accuracy of peak positions is defined as +/- 0.2 degrees due to experimental differences like instrumentations, and sample preparations.

### Example 1: Preparation of crystalline form of PAL-HCl characterized by data selected from the group consisting of:a PXRD with peaks at about 13.0, 15.4 and 17.5 degrees two-theta, a PXRD pattern substantially as depicted in figure 1, and combination thereof.

A solution of about 3.5 g of palonosetron hydrochloride diastereoisomeric mixture (60.5% 3aS isomer, 39.6% 3aR isomer) in 90 ml of MeOH was concentrated to 12 mL of residual volume under vacuum. 38 mL of a mixture isopropanol-water 97:3 were added at reflux temperature leading to a solution. The solvent was evaporated at atmospheric pressure to 28 mL residual volume in order to eliminate most of the residual MeOH. To the suspension further 28 mL of mixture isopropanol-water 97:3 were added at reflux temperature to achieve complete dissolution. The solution was cooled in 100 minutes to 50°C, leading to the seeding of the solid. The mixture was concentrated under vacuum at 50°C until 34 mL of residual volume. The suspension was cooled in 30' to 20°C, and after 30' of stirring it was filtrated. The solid was dried in a nitrogen/vacuum oven at 70°C for 10 hours, leading to a crystalline diastereoisomeric mixture of palonosetron HCl of 96% 3aS isomer and 4% 3aR isomer.

### Example 2: Preparation of crystalline form of PAL-HCl characterized by data selected from the group consisting of:a PXRD with peaks at about 13.0, 15.4 and 17.5 degrees two-theta, a PXRD pattern substantially as depicted in figure 1, and combination thereof.

A solution of about 4 g of palonosetron hydrochloride diastereoisomeric mixture (59.4% 3aS isomer, 40.6% 3aR isomer) in 92 ml of MeOH was concentrated to 12 mL of residual volume under vacuum. 40 mL of isopropanol were added at reflux temperature. The solvent was evaporated at atmospheric pressure to 32 mL residual volume in order to eliminate most of the residual MeOH. To the suspension further 12 mL of isopropanol was added, and the mixture was evaporated to 32 mL residual volume. At reflux temperature 1.6 mL of water were added, leading to a mixture 95:5 of isopropanol-water. Further 4 mL of mixture 95:5 of isopropanol-water were added to achieve dissolution at reflux temperature. The solution was cooled to 0°C in 300 minutes (crystallization occurred at 48°C). After 18 hours of stirring at 0°C the solid was filtered off leading to 1.7 g of a crystalline diastereoisomeric mixture of palonosetron HCl of 96.7% 3aS isomer and 3.3% 3aR isomer.

### Example 3: Preparation of crystalline form of PAL-HCl characterized by data selected from the group consisting of: a PXRD with peaks at about 13.0, 15.4 and 17.5 degrees two-theta, a PXRD pattern substantially as depicted in figure 1, and combination thereof.

A solution of about 4 g of palonosetron hydrochloride diastereoisomeric mixture (59.4% 3aS isomer, 40.6% 3aR isomer) in 92 ml of MeOH was concentrated to 12 mL of residual volume under vacuum. 40 mL of isopropanol were added at reflux temperature. The solvent was evaporated at atmospheric pressure to 32 mL residual volume in order to eliminate most of the residual MeOH. To the suspension further 12 mL of isopropanol evaporating to 32 mL residual volume. At reflux temperature 0.96 mL of water were added, leading to a mixture 97:3 of isopropanol-water. Further 8 mL of mixture 97:3 of isopropanol-water were added to achieve dissolution at reflux temperature. The solution was cooled to 0°C in 300 minutes (crystallization occurred at 52°C). After 18 hours of stirring at 0°C the solid was filtered off leading to 1.75 g of a crystalline diastereoisomeric mixture of palonosetron HCl of 96.1% 3aS isomer and 3.9% 3aR isomer.

### Example 4: Preparation of crystalline form of PAL-HCl characterized by data selected from the group consisting of: a PXRD with peaks at about 13.0, 15.4 and 17.5 degrees two-theta, a PXRD pattern substantially as depicted in figure 1, and combination thereof.

A solution pf about 4 g of palonosetron hydrochloride diastereoisomeric mixture (63% 3aS isomer, 37% 3aR isomer) in 170 ml of MeOH was concentrated to solid residual under vacuum at the rotary evaporator. 80 mL of isopropanol and 4 mL of water were added and the suspension was heated to reflux temperature. The solvent was evaporated at atmospheric pressure to 32 mL residual volume in order to eliminate most of the residual MeOH. To the suspension further 12 mL of isopropanol evaporating to 32 mL residual volume. At reflux temperature 0.96 mL of water were added, leading to a mixture 97:3 of isopropanol-water. Further 8 mL of mixture 97:3 of isopropanol-water were added to achieve dissolution at reflux temperature. The solution was cooled to 0°C in 300 minutes. After 18 hours of stirring at 0°C the solid was filtered off leading to 1.75 g of a crystalline diastereoisomeric mixture of palonosetron HCl of 96.1% 3aS isomer and 3.9% 3aR isomer.

### Example 5: Preparation of crystalline form of PAL-HCl characterized by data selected from the group consisting of: a PXRD with peaks at about 12.1, 15.8 and 17.3 degrees two-theta, a PXRD pattern substantially as depicted in figure 2, and combination thereof.

1.1 g of Palonosetron hydrochloride diastereoisomeric mixture (97.7% 3aS isomer, 1.5% 3aR isomer) solid dried on the filter funnel under nitrogen was suspended in 33 ml of mixture 95:5 isopropanol-water. The suspension was heated to reflux temperature until dissolution, then cooled to 60°C in 100', leading to crystals formation, then cooled to 40°C in further 45', then cooled to 20°C in 30' and stirred at this temperature overnight. The solid was filtered off and dried for 18 h at 70°C in a nitrogen/vacuum oven leading to 0.56 g of a crystalline diastereoisomeric mixture of palonosetron HCl of 99.7% 3aS isomer and 0.3% 3aR isomer.

### Example 6: Preparation of crystalline form of PAL-HCl characterized by data selected from the group consisting of: a PXRD with peaks at about 12.1, 15.8 and 17.3 degrees two-theta, a PXRD pattern substantially as depicted in figure 2, and combination thereof.

1.5 g of Palonosetron hydrochloride diastereoisomeric mixture (96% 3aS isomer, 4% 3aR isomer) solid dried on the filter funnel under nitrogen was suspended in 30 ml of mixture 95:5 isopropanol-water. The suspension was heated to reflux temperature until dissolution, then cooled to 45°C in 60', then cooled to 20°C in further 60', then cooled to 0°C in 30' and stirred at this temperature for 30'. The solid was filtered off and dried for 18 h at 70°C in a nitrogen/vacuum oven leading to 0.78 g of a crystalline diastereoisomeric mixture of palonosetron HCl of 99.6% 3aS isomer and 0.4% 3aR isomer.

### Example 7: Preparation of crystalline form of PAL-HCl characterized by data selected from the group consisting of: a PXRD with peaks at about 12.1, 15.8 and 17.3 degrees two-theta, a PXRD pattern substantially as depicted in figure 2, and combination thereof.

3.43 g of Palonosetron hydrochloride diastereoisomeric mixture (99.6% 3aS isomer, 0.4% 3aR isomer) solid dried on the filter funnel under nitrogen was suspended in 68 ml of mixture 95:5 isopropanol-water. The suspension was heated to reflux temperature until dissolution, then cooled to 50°C in 100'(crystals formation was observed at 59°C). The suspension was concentrated to 32 mL of residual volume at this temperature under vacuum. The suspension was then cooled to 20°C in 60' and stirred at this temperature for 60'. The solid was filtered off and dried for 18 h at 70°C in a nitrogen/vacuum oven leading to 2.6 g of a crystalline diastereoisomeric mixture of palonosetron HCl of 99.9% 3aS isomer and 0.1% 3aR isomer.

### Example 8: Preparation of the free base of Cp 9563 using thionyl chloride

### Preparation of Cp 9588:

In a 250 mL glass reactor, under nitrogen atmosphere, 0.24 g of DMF, 310 mL of toluene and 44.8 g of tetrahydronaphthoic acid were charged at room temperature. The suspension was heated to 51±2°C and 32 g of thionyl chloride were added drop-wise in about 60 minutes. The addition involves the evolution gas.
The solution was stirred at 51±2°C for 60 minutes. The solvent was then distilled off at reduced pressure with internal temperature 46±2°C until 210 mL of residual volume (5.1 volumes vs tetrahydronaphtoic acid). The yellowish solution of acyl chloride thus obtained was used with no further purification nor isolation in the following step.

### Reacting the salt of Cp 9771 and Cp 9588:

In a 1000 mL glass reactor, under nitrogen atmosphere, 29.6 g of NaOH pellets and 50 mL of water were charged. The dissolution is highly exothermic. The fluid in the reactor jacket was cooled in order to keep the internal temperature at about 60°C. To the solution, at 59±2°C, 270 mL of toluene, 42 g of aminoquinuclidine 2HCl were added. The suspension was stirred at 60±2°C for 60 minutes.
To the suspension, at 60 ±2°C, the toluenic solution of acyl chloride obtained in step 1 was added dropwise in about 1 hour. After 30 minutes the reaction was considered complete, thus 200 mL of water were added.
120 ml of toluene were then loaded. The suspension was stirred at 53±2°C for 30 minutes, then the phases were separated. The aqueous phase was eliminated and the organic one was washed with 200 mL of a solution of K₂CO₃ 5% in H₂O (prepared dissolving 10 g of K₂CO₃ in about 190 ml of H₂O).
The organic phase was concentrated under reduced pressure, with internal temperature 55±2°C until 180 mL of residual volume.

### Example 9: Preparation of Cp 9558 from the free base of Cp 9563

In a 500 mL glass reactor, under nitrogen atmosphere, 15 g of aminoquinuclidin naphtalen amide and 200 mL of THF were charged at room temperature. The solution was cooled to -25±2°C.
By keeping the temperature below -15 ±2°C, 54 mL of the solution of hexyl lithium in hexane were added drop-wise in 30 min.
The violet solution was stirred for 20 minutes at T=-20±2°C, then the temperature was lowered to -30±2°C and 5.9 g of DMF were added in 30 min keeping the temperature below -25 °C. The yellowish solution was stirred for 60 minutes at - 25 ±2°C.
To the solution heated to 0±2°C were then added 15 mL of water and 28.5 g of HCl 37% keeping the temperature below 15°C. After 40 minutes of stirring 120 mL of water were loaded and the mixture was stirred for 20 min at room temperature. The phases were then separated and 130 mL of DCM were added to the aqueous phase containing the hydrochloric salt of the 3-3a dehydro Palonosetron.
To the mixture 28.3 g of a solution of NaOH 15% w/w in water were added (prepared dissolving 4.25 g of NaOH pellets in 24 mL of water). The final pH was 10.5.
The layers were separated and 50 mL of a solution of NaCl 15% w/w in water (prepared dissolving 7.5 g of NaCl pellets in about 42.5 mL of water) were added to the organic phase.
The phases were separated and to the organic one 150 mL of 2-propanol and 6.3 g of HCl 37% were added.
The solvent was distilled off under reduced pressure until 90 mL of residual volume with internal T between 35 and 45°C.
The suspension was heated to 50±2°C and 100 mL of n-heptane were charged at this temperature.
The suspension was cooled to 0±2°C in 120 minutes, then after 30 minutes of stirring at this temperature the solid was filtered off washing the cake with a mixture of 15 mL of IPA and 15 mL of n-heptane pre-cooled to 0±2°C.
The solid was dried in oven under vacuum at 70±2°C for 18 hours, leading to 15.0 g of 3-3a dehydro Palonosetron HCl. HPLC purity about 98%.

### Example 10: Preparation of Palonosetron hydrochloride from Cp 9588 in methanol.

In a 500 mL glass autoclave 8 g of Palonosetron 3-3a-dehydro HCl and 150 mL of MeOH were charged. 0.8 g of Pd 10% on Carbon, at 50% humidity, were added. H₂ at 4-5 bar was charged.
The suspension was heated to 50°C and stirred in these conditions for 21 hours (adding H₂ when internal pressure <3 bar). The suspension was then cooled to 5±2°C and 2.56 g of solution of SO₂ 5% in water were added to quench the reaction.
After 30 minutes of stirring at this temperature the suspension was filtered over a cake of 10 g of Tecnolite Special 1 washing with 100 mL of MeOH.
(79°C) until dissolution, then cooled to 0±2°C in 300 minutes. After 30' of stirring at 0±2°C the solid was filtered off and the cake was washed with 20 mL of 2-propanol pre-cooled to 0±2°C. The solid was squeezed on the filter funnel for 1 hour.
3 g of wet Palonosetron HCl were obtained as white solid. HPLC purity about 98.8% The wet solid was dried at 70°C for a 18 hours under vacuum, leading to 2.9 g of Palonosetron HCl.

### Example 11: Preparation of crystalline form of PAL-HCl characterized by data selected from the group consisting of: a PXRD with peaks at about 12.1, 15.8 and 17.3 degrees two-theta, a PXRD pattern substantially as depicted in figure 2, and combination thereof.

100 mg of crystalline form of PAL-HCl characterized by data selected from the group consisting of: a PXRD with peaks at about 13.0, 15.4 and 17.5 degrees two-theta, a PXRD pattern as depicted in figure 1, and combination thereof was stored at 100% relative humidity at room temperature for 1 week. The polymorphic form of the material was measured by X-ray powder diffraction after 1 week storage.

### Example 12: Preparation of crystalline form of PAL-HCl characterized by data selected from the group consisting of: a PXRD with peaks at about 13.0, 15.4 and 17.5 degrees two-theta, a PXRD pattern substantially as depicted in figure 1, and combination thereof

5 g of Palonosetron HCl (99.6% 3aS isomer) dry were dissolved in 150 g of MeOH at 25°C. The solvent was evaporated on the rotary evaporator at 25°C (bath temperature) until dry residual. The solid was dried under vacuum at 70°C leading to 4.80 g of product.

Further aspects and features of the present invention are set out in the following numbered clauses.
1. A crystalline form of Palonosetron hydrochloride (PAL-HCl), characterized by data selected from the group consisting of: a PXRD having peak reflections at about 13.0, 15.4 and 17.5±0.2 degrees two-theta, a PXRD pattern as depicted in figure 1, and a combination thereof.
2. The crystalline form of PAL-HCl of clause 1, characterized by a PXRD having peak reflections at about 13.0, 15.4 and 17.5 ±0.2 degrees two-theta.
3. The crystalline form of PAL-HCl of any one of clauses 1 or 2, characterized by a PXRD pattern as depicted in figure 1.
4. The crystalline form of PAL-HCl of any one of the preceding clauses, further characterized by PXRD peak reflections at about 7.1, 13.7, 14.2, 16.2, 18.5, 20.0 and 22.1 ±0.2 degrees two-theta.
5. The crystalline form of PAL-HCl of any one of the preceding clauses, wherein the crystalline form has a DSC thermogram having an endothermic peak at about 311 °C.
6. The crystalline form of PAL-HCl of any one of the preceding clauses, wherein the crystalline form has a weight loss of less than about 0.1% measured at temperatures less or equal to 153°C by TGA.
7. The crystalline form of PAL-HCl of any one of the preceding clauses, wherein the crystalline form is an anhydrous form of PAL-HCl.
8. The crystalline form of PAL-HCl of any one of the preceding clauses, wherein the crystalline form has less than 20% crystalline form of Palonosetron hydrochlroide characterized by data selected from the group consisting of: a PXRD having peak reflections at about 12.1, 15.8 and 17.3 ±0.2 degrees two-theta, a PXRD pattern as depicted in figure 2, and a combination thereof
9. A process preparing crystalline form of PAL-HCl according to any preceding clause, comprising crystallizing Palonosetron hydrochloride having less than 65% of 3aS Palonosetron HCl of the following formula, 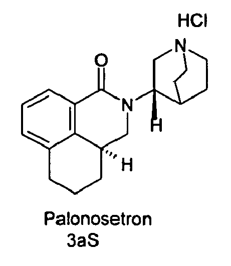 from a solvent selected from the group consisting of: methanol, a mixture of isopropanol (IPA) and water, and a mixture of methanol, isopropanol and water at a temperature below 55°C.
10. The process of clause 9, comprising;
   a) providing a solution of PAL-HCl having less than 65% of 3aS Palonosetron HCl in a solvent selected from the group consisting of: methanol, a mixture of isopropanol and water, and a mixture of methanol, isopropanol and water, and
   b) precipitating said crystalline PAL-HCl at a temperature below 55°C.
11. The process of clause 10, wherein the palonosteron HCl in step (a) has about 50% to about 65% of the 3aS Palonosetron HCl isomer.
12. The process of any one of clauses 10 or 11, wherein the solution in step (a) is prepared by a process comprising combining PAL-HCl having less than 65% of 3aS Palonosetron HCl and the solvent at a temperature of about 25°C to about reflux.
13. The process of any one of clauses 10 to 12, wherein a solution of PAL-HCl in methanol is provided by combining PAL-HCl having less than 65% of 3aS Palonosetron HCl and methanol at a temperature of about 25°C.
14. The process of any one of clauses 10 to 12, wherein a solution of PAL-HCl in a mixture of IPA and water is provided by combining PAL-HCl having less than 65% of 3aS Palonosetron HCl and a mixture of IPA and water at a temperature of at least 75°C.
15. The process of any of clauses 10 to 12, wherein the solution of PAL-HCl in a mixture of methanol, IPA and water is provided by combining a methanolic residue of PAL-HCl having less than 65% of 3aS Palonosetron HCl with a mixture of IPA and water at a temperature of at least 75°C.
16. The process of clause 15, wherein the methanolic residue of PAL-HCl having less than 65% of 3aS Palonosetron HCl is prepared by a process comprising
   (a) concentrating a solution of PAL-HCl having less than 65% of 3aS Palonosetron HCl in methanol,
   (b) adding IPA or a mixture of IPA and water to obtain a mixture, and
   (c) concentrating the mixture to obtain the said methanolic residue.
17. The process of any one of clauses 14 to 16, wherein the ratio of the solvents in the mixture of IPA and water is about 94:6 to about 97:3.
18. The process of clause 17, wherein the ratio is about 95:5 to about 97:3.
19. The process of any one of clauses 14 to 18, wherein combining PAL-HCl having less than 65% of 3aS Palonosetron HCl or a methanolic residue thereof with a mixture of IPA and water is done at about 75°C to about 90°C.
20. The process of any one of clauses 14 to 19, wherein the solution comprises methanol, IPA and water in a ratio of about 0.5:94.5:5 to about 0.1:94.9:5 VN, respectively.
21. The process of any one of clauses 10 to 20, wherein precipitation comprises the following steps
   (a) cooling the obtained solution,
   (b) evaporating the solvent or
   (c) by performing both steps (a) and (b),
   wherein steps (a)-(c) are done at a temperature below 55°C.
22. The process of any one of clauses 10 to 13, wherein methanol is the solvent and precipitation is achieved by evaporating the solution at a temperature of about 25°C.
23. The process of any one of clauses 10-12 and 14, wherein a mixture of IPA and water is the solvent and precipitation is achieved by cooling the solution to a temperature below about 55°C to obtain a suspension.
24. The process of clause 23, wherein cooling is to a temperature of about 50°C to about 0°C.
25. The process of any of Clauses 9 to 24, further comprising recovering the obtained crystalline form of PAL-HCl.
26. A crystalline form of PAL-HCl, characterized by data selected from the group consisting of: a PXRD having peak reflections at about 12.1, 15.8 and 17.3 ±0.2 degrees two-theta, a PXRD pattern as depicted in figure 2, and a combination thereof.
27. The crystalline form of PAL-HCl of clause 26, characterized by a PXRD having peak reflections at about 12.1, 15.8 and 17.3 ±0.2 degrees two-theta.
28. The crystalline form of PAL-HCl of any one of clauses 26 or 27, characterized by a PXRD pattern as depicted in figure 2.
29. The crystalline form of PAL-HCl of any one of clauses 26 to 28, further characterized by PXRD peak reflections at about 7.1, 13.8, 14.2, 14.5, 18.5, 20.0 and 30.3 ±0.2 degrees two-theta.
30. The crystalline form of PAL-HCl of any one of clauses 26 to 29, wherein the crystalline form has a DSC thermogram having an endothermic peak at about 313°C.
31. The crystalline form of PAL-HCl of any one of clauses 26 to 30, wherein the crystalline form has a weight loss of less than about 0.1 % measured at temperatures less or equal to 152°C by TGA.
32. The crystalline form of PAL-HCl of any one of clauses 26 to 31, wherein the crystalline form is an anhydrous form of PAL-HCl.
33. The crystalline form of PAL-HCl of one of clauses 26 to 32, wherein the crystalline form has less than 20% of the crystalline form of Palonosetron hydrochlroide characterized by data selected from the group consisting of: a PXRD having peak reflections at about 13.0, 15.4 and 17.5 ±0.2 degrees two-theta, a PXRD pattern as depicted in figure 1, and a combination thereof.
34. A process preparing a crystalline form of PAL-HCl according to any of clauses 26 to 33, comprising crystallizing Palonosetron hydrochloride having at least 96% of 3aS Palonosetron HCl of the following formula, 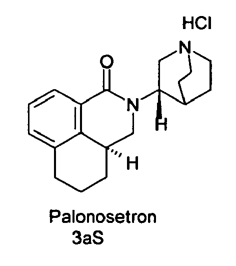 from a mixture of isopropanol and water at a temperature above 55°C.
35. The process of clause 34, wherein the crystallization is carried out by a process comprising
   (a) providing a solution of PAL-HCl having at least 96% of 3aS Palonosetron HCl in a mixture comprising IPA and water at a temperature above 55°C, and
   (b) precipitating the said crystalline PAL-HCl by cooling the solution at a temperature of at least about 55°C.
36. The process of clause 35, wherein the solution in step (a) is provided by a process comprising admixing PAL-HCl having at least 96% of 3aS Palonosetron HCl with a mixture comprising IPA and water to obtain a suspension, and heating the suspension to a temperature above 55°C.
37. The process of any one of clauses 34 to 36, wherein the ratio of the solvents in the mixture of IPA-water is of about 94:6 to about 98:2.
38. The process of clause 37, wherein the ratio of the solvents in the mixture of IPA-water is of about 95:5 to about 98:2.
39. The process of any one of clauses 36 to 38, wherein the suspension is heated to a temperature of at least 75°C.
40. The process of clause 39, wherein the suspension is heated to about 75°C to about 90°C.
41. The process of any one of clauses 35 to 40, wherein the precipitation is achieved by cooling the solution to a temperature of at least about 55°C to obtain a suspension.
42. The process of clause 41, wherein the solution is cooled to a temperature of about 58°C to about 60°C.
43. The process of any of clauses 34 to 42, further comprising recovering the obtained crystalline form of PAL-HCl.
44. A process for preparing the crystalline Palonosetron hydrochloride of any of clauses 26 to 33 comprising exposing the crystalline Palonosetron hydrochloride as defined in any of clauses 1 to 8 to relative humidity of about 100%.
45. The process of clause 44, wherein the exposure is at a temperature of about 20°C to about 30°C.
46. The process according to any one of clauses 44 or 45, wherein the exposure is for a period of about 2 days to about 10 days.
47. A pharmaceutical composition comprising a crystalline form of Palonosetron hydrochloride as defined in any of clauses 1 to 8 and/or clauses 26 to 33 and a pharmaceutically acceptable excipient.
48. A process for preparing a pharmaceutical composition according to clause 47 comprising combining a crystalline form of Palonosetron hydrochloride as defined in any of Clauses 1 to 8 and/or clauses 26 to 33 and a pharmaceutically acceptable excipient.

## Claims

1. A crystalline form of palonosetron hydrochloride (PAL-HCl), **characterized by** data selected from the group consisting of: a PXRD having peak reflections at about 12.1, 15.8 and 17.3 ±0.2 degrees two-theta, a PXRD pattern as depicted in figure 2, and a combination thereof.

2. The crystalline form of PAL-HCl of claim 1, **characterized by** a PXRD having peak reflections at about 12.1, 15.8 and 17.3 ±0.2 degrees two-theta.

3. The crystalline form of PAL-HCl of claim 1, **characterized by** a PXRD pattern as depicted in figure 2.

4. The crystalline form of PAL-HCl of any preceding claim, further **characterized by** PXRD peak reflections at about 7.1, 13.8, 14.2, 14.5, 18.5, 20.0 and 30.3 ±0.2 degrees two-theta.

5. The crystalline form of PAL-HCl of any preceding claim, wherein the crystalline form has a DSC thermogram having an endothermic peak at about 313°C.

6. The crystalline form of PAL-HCl of any preceding claim, wherein the crystalline form has a weight loss of less than about 0.1 % measured at temperatures less or equal to 152°C by TGA.

7. The crystalline form of PAL-HCl of any preceding claim, wherein the crystalline form is an anhydrous form of PAL-HCl.

8. The crystalline form of PAL-HCl of any preceding claim, wherein the crystalline form has less than 20% of the crystalline form of PAL-HCl **characterized by** data selected from the group consisting of: a PXRD having peak reflections at about 13.0, 15.4 and 17.5 ±0.2 degrees two-theta, a PXRD pattern as depicted in figure 1, and a combination thereof.

9. The crystalline form of PAL-HCl of any preceding claim having a purity by HPLC of about 98.8%.

10. The crystalline form of PAL-HCl of any preceding claim comprising 99.7% of the 3aS isomer and 0.3% of the 3aR isomer.

11. The crystalline form of PAL-HCl of any preceding claim comprising 99.9% of the 3aS isomer and 0.1 % of the 3aR isomer.

12. A pharmaceutical composition comprising a crystalline form of PAL-HCL as defined in any of claims 1 to 11 and a pharmaceutically acceptable excipient.

13. A process for preparing a pharmaceutical composition according to claim 12 comprising combining a crystalline form of PAL-HCL as defined in any of Claims 1 to 11 and a pharmaceutically acceptable excipient.
